# EUROPEAN PATENT APPLICATION

(11) **EP 0 791 369 A1**
(43) Date of publication of application: **27.08.1997**
(21) Application number: 97101027.7
(22) Date of filing: 23.01.1997
(51) Int. Cl.: A61M 5/142, F04B 43/08

(54) **Pump**

(30) Priority: 22.02.1996 SE 9600659
(71) Applicant: Siemens-Elema AB, 171 95 Solna 1 (SE)
(72) Inventor: Sjöholm, Gösta, 178 34 Ekerö (SE)

(57) **Abstract**

A pump for viscous substances comprises a pump chamber (1) with an inlet (18), intended for connection to a source of the substance to be pumped, and an outlet (12), equipped with a valve, for pumping out the substance sucked into the pump chamber during an intake phase. The pumping action is achieved by varying the volume of the pump chamber. A unit is arranged to control variations in the volume of the pump chamber so the delivery phase is considerably shorter than the intake phase. The inlet is further devised so it presents a greater resistance to flow than the outlet when the outlet valve (7) is open, so backflow of substance through the inlet during the delivery phase is only corresponds to a fraction of the stroke volume of the pump.

## Description

The present invention relates to a pump for viscous substances, said pump comprising a pump chamber with an inlet, intended for connection to a source of the substance to be pumped, and an outlet, equipped with a valve, for pumping out the substance sucked into the pump chamber during an intake phase, pumping action being achieved by varying the volume of the pump chamber.

A pump of this kind is previously known through DE,A1,33 20 443. Micropumps such as these are intended for pumping small, exact flows of viscous substances, such as liquid medication in implantable medication pumps.

For safety reasons, the medication reservoir in certain kinds of medication pumps is kept at a sub-ambient pressure. See US,A,4 191 181. The pump must then be capable of pumping liquid from this area of low pressure into the pump chamber. This has proved to be difficult with pumps made using micromechanical technics.

The current type of pumps must also display reliable valve operation and an ability to pump gas bubbles (air) out of the low pressure reservoir. These requirements can be achieved with a pump according to US,A,4 808 089. The disadvantage of these pumps is that they are expensive to manufacture, since a plurality of parts to be assembled must be manufactured with very close tolerances.

The object of the present invention is to eliminate these disadvantages of prior art pumps and simplify construction, so the pumps can be mass-produced at low cost, by efficient methods, with simultaneous retention of their ability to pump very small flows with great accuracy and improved operating reliability.

This purpose is achieved with a pump of the kind stated in the introduction with the characteristics set forth in claim 1.

With the pump according to the invention, the need for an inlet valve is eliminated, thereby increasing operating reliability. The pump can suitably be made of silicon using etching methods. The pump can then be made very small with a very small chamber volume. The pump can also be advantageously constructed, so its dead space is substantially equal to zero when the pump in its operating cycle is in a position at which the pump chamber has the smallest volume, and very small volumes can be pumped with great accuracy.

According to advantageous embodiments of the pump according to the invention, the increased flow resistance in the inlet can be realized with restriction or constriction of the inlet, e.g. by devising the inlet as a relatively long and narrow channel to reduce space requirements, or as a capillary. This would accordingly reduce the backflow of substance during the pump's compression or delivery phase. The inlet can advantageously be devised so backflow of substance through the inlet during the delivery phase is less than 5% of the stroke volume of the pump.

The pumping function is achieved by varying the volume of the pump chamber and, according to advantageous embodiments of the pump according to the invention, one wall of the pump chamber is moveable, and the volume of the pump chamber can be varied by moving the said wall. The pump chamber can consist of e.g. a cylinder with a moveable piston therein, the cylinder being made of a material, such as a piezoelectric ceramic or a magnetostrictive material which axially contracts when electrically acted upon.

According to additional advantageous embodiments of the pump according to the invention, the inlet valve is arranged to be opened by the pressure gradient produced across the valve during the pump stroke or in the delivery phase. Or the outlet valve can be devised with a separate, maneuverable opening mechanism.

According to other advantageous embodiments of the pump according to the invention, the pump is made of a ferromagnetic material or contains a core of ferromagnetic material, a permanent magnet or an electromagnet being simultaneously arranged on the exterior of or interior of one cylinder end to keep the piston next to this end and return the piston after a pump stroke. Or the end can be made of a permanently magnetic material.

The pump stroke is achieved by the discharge of current in a solenoid.

One exemplifying embodiment of the pump according to the invention will now be described in greater detail, referring to the enclosed drawing.

The embodiment of the pump according to the invention shown in the figure comprises a cylinder 3 with tightly sealed ends 24 and 15. A piston 20 is arranged in the cylinder 3 next to the end 24. The volume of the cylinder is essentially the same as the volume of the piston 20 plus the volume of the pump chamber 1, the latter being essentially equal to the pump's stroke volume.

The piston 20 is arranged with play 3 in the cylinder so the piston can move freely in same.

The cylinder 3 is made of a material which contracts on stimulation, thereby causing the cylinder 3 to contract axially. This material is a piezoelectric ceramic or a magnetostrictive material with a property causing it to contract axially when subjected to electrical stimulation. The material's contraction is such that the contraction of the cylinder 3 is equal to or somewhat greater than the volume of the pump chamber, i.e. the pump's stroke volume according to the above.

The piston 20 is made of a ferromagnetic material or has a core of ferromagnetic material encased in some other material. The end 24 serves as a seal closing off the cylinder 3 from the exterior, and a permanent magnet or electromagnet is arranged on the exterior of the end to hold the piston 20 next to this end 24 and return the piston 20 to the resting position shown in the FIGURE after a pump stroke has been performed by contraction of the cylinder 3, as described above. The magnet can also be arranged inside the end 24, or the end 24 can be made of a permanently magnetic material.

An outlet with an outlet valve 7 is arranged in the end 15. The one-way valve is closed in the normal or resting position. The valve 7 is retained in its resting position by a spring, schematically depicted at 22 in the figure, or by a magnetic force. During the delivery phase or pump stroke, the positive pressure produced in the pump chamber 1 overcomes the spring force or magnetic force, and the valve 7 opens. The outlet valve 7 closes after each pump stroke. A valve with separate, active control can also be used. The valve 7 is further devised to minimize the pump chamber's 1 dead space.

An inlet 18 to the pump chamber 1 is arranged near the outlet valve 7. The inlet 18 can open into e.g. the joint between the end 15 and the end of the cylinder, but other locations for the inlet are obviously possible. The inlet 18 is devised as a capillary or a relatively long, narrow channel, i.e. the inlet 18 is devised to present great resistance to flow than the outlet with the outlet valve 7 open. The inlet 18 is further devised and made of a material so it remains coated with a film of fluid inside the capillary at all times.

Stimulation of the cylinder wall is controlled in such a way that the piston 20 moves rapidly during the pump stroke, i.e. the pump stroke is short. As a result of the great resistance to flow in the inlet 18, only a small fraction, preferably less than 5%, of all the delivered substance pumped passes through the inlet 18. After each pump stroke, the piston 20 is slowly drawn back to the normal position by the magnetic force exerted by the end 24 when the cylinder 3 resumes its original dimensions. This process is relatively slow, giving the pump chamber 1 time to fill with the substance to be pumped through the inlet 18, despite the inlet's great resistance to flow, during this relatively long intake phase during which the outlet valve 7 is closed. As a result of this slow intake phase, cavitation is avoided to the greatest extent possible during the intake or filling phase.

So the pump's operation is briefly as follows:

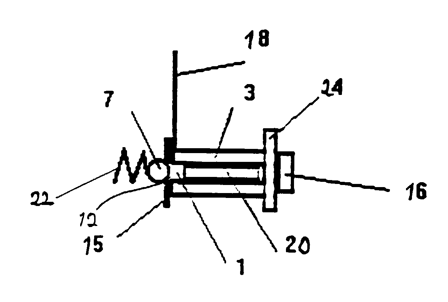

Electrical stimulation causes the cylinder 3 to contract rapidly, forcing the piston 20 to its end position to the left in FIG. 1 and reducing the volume of the pump chamber 1 to zero. The outlet valve 7 is opened by the pressure generated in the pump chamber or by a separate operation, and fluid remaining in the pump chamber 1 is pumped out, mainly through the outlet.

Stimulation then ends, and the cylinder 3 returns to its normal state. Here, the piston 20 follows the movement of the end 24 as a result of the magnetic force. This process takes place slowly so new fluid has time to be drawn into the pump chamber 1, without cavitation, and the next pump cycle can start thereafter.

The pump according to the invention as described above can be advantageously manufactured of silicon and made very small and with a very small chamber volume, by means of micromechanical technics or etching operations, with a very small chamber volume. Moreover, manufacturing costs can be kept low. The pump is further suitable for pumping very small flows with great accuracy, therefore making it suitable for use in medical implants.

## Claims

1. A pump for viscous substances comprising a pump chamber with an inlet, intended for connection to a source of the substance to be pumped, and an outlet, equipped with a valve, for pumping out the substance sucked into the pump chamber during an intake phase, pumping action being achieved by varying the volume of the pump chamber, **characterized in** that a unit is arranged to control variations in the volume of the pump chamber, so the delivery phase is considerably shorter than the intake phase, and in that the inlet is devised so it presents a greater resistance to flow than the outlet when the outlet valve is open, so the backflow of substance through the inlet during the delivery phase only corresponds to a fraction of the stroke volume of the pump.

2. The pump according to claim 1, **characterized in** that the inlet is devised with a restriction or constriction in order to achieve the said increased resistance to flow.

3. The pump according to claim 1, **characterized in** that the inlet is devised as a relatively long, narrow channel, or capillary, in order to achieve the said increased resistance to flow.

4. The pump according to claim 3, the inlet being devised as a capillary, **characterized in** that the capillary is made of a material which always remains coated with a film of the pumped substance.

5. The pump according to any one of the claims 1-4, **characterized in** that the inlet is so devised that the backflow of substance through the inlet during the delivery phase is less than 5% of the stroke volume of the pump.

6. The pump according to any one of the claims 1-5, **characterized in** that one wall of the pump chamber is moveable, and the volume of the pump chamber can be varied by moving the said wall.

7. The pump according to any one of the claims 1-6, **characterized in** that the pump chamber consists of a cylinder and a moveable piston arranged therein, arranged at one end of the cylinder, and the cylinder is made of a material which axially contracts when electrically acted upon.

8. The pump according to claim 7, **characterized in** that the cylinder is made of an piezoelectric ceramic or a magnetostrictive material.

9. The pump according to claim 7 or 8, **characterized in** that the inlet is arranged between the end and the wall of the cylinder at the end of the cylinder opposite the moveable end.

10. The pump according to any one of the claims 7-9, **characterized in** that the outlet is arranged at the end of the cylinder opposite the moveable end.

11. The pump according to any one of the claims 1-10, **characterized in** that the outlet valve is arranged to be opened by the pressure gradient across the valve during the delivery phase.

12. The pump according to any one of the claims 1-10, **characterized in** that the outlet valve is devised with a separate maneuverable opening mechanism.

13. The pump according to any one of the claims 7-12, **characterized in** that the piston is made of a ferromagnetic material or has a core made of a ferromagnetic material.

14. The pump according to claim 13, **characterized in** that a permanent magnet or electromagnet is arranged on the exterior of or in the interior of the said end in order to hold the piston and return it after each pump stroke.

15. The pump according to claim 13, **characterized in** that the said end is made of a permanently magnetic material in order to hold and return the piston after each piston stroke.

16. The pump according to any one of the claims 1 - 15, **characterized in** that it is made of silicon.
